# EUROPÄISCHE PATENTSCHRIFT

(11) **EP 0 871 424 B1**
(45) Veröffentlichungstag und Bekanntmachung des Hinweises auf die Patenterteilung: **28.08.2002**
(21) Anmeldenummer: 96944015.5
(22) Anmeldetag: 19.12.1996
(51) Int. Cl.: A61K 7/00

(54) **HAARFÄRBEMITTEL UND ANWENDUNGSMISCHUNG ZUM FÄRBEN VON HUMANHAAREN**
HAIR COLORANT AND APPLICATION MIXTURE FOR COLOURING HUMAN HAIR
COLORANT POUR CHEVEUX ET MELANGE D'APPLICATION POUR COLORER DES CHEVEUX NATURELS

(30) Priorität: 05.01.1996 DE 19600221
(43) Veröffentlichungstag der Anmeldung: 21.10.1998
(73) Patentinhaber: Hans Schwarzkopf GmbH & Co. KG, 22763 Hamburg (DE)
(72) Erfinder: AKRAM, Mustafa, D-22457 Hamburg (DE); WOLFF, Wolfgang, D-22941 Bargteheide (DE); ROHWEDER, Sandra, D-22763 Hamburg (DE); SCHWARTZ, Stephan, D-22880 Wedel (DE); KLEEN, Astrid, 40699 Erkrath (DE)
(74) Vertreter: Foitzik, Joachim Kurt
(86) Internationale Anmeldenummer: EP9605714
(87) Internationale Veröffentlichungsnummer: WO97025017

(56) Entgegenhaltungen:
- DE-A- 4 233 874
- FR-A- 2 510 401
- US-A- 4 183 366
- US-A- 4 275 055
- US-A- 4 658 839

## Beschreibung

Die Erfindung betrifft feste Haarfärbemittel sowie daraus hergestellte Anwendungsmischungen zum Färben von Haaren.

Die Verwendung von Pflanzenfarbstoffen in Haarfärbemitteln ist lange bekannt. Neuere Entwicklungen auf diesem Färbegebiet sind beispielsweise in der FR-OS 1 583 606, der BE-OS 900219, der GB-OS 2 190 104 und der DE-PS 38 29 102 offenbart. Der DE-A1 42 33 874 sind Haartönungsmittel in Granulatform zu entnehmen, die Pflanzenfarbstoffe in Kombination mit synthetischen direktziehenden Farbstoffen enthalten.

Diese bekannten Färbemittel auf Basis von Pflanzenfarbstoffen haben allerdings den Nachteil, daß die damit erzielte Abdeckung von grauen Haaren in vielen Fällen nicht befriedigt.

Andererseits ist dem Fachmann das Färben mit sogenannten Oxidationsfarbstoffen bekannt. Hierbei wird die Färbung dadurch erzielt, daß Oxidationsfarbstoffvorprodukte in das Haar eindringen und dort unter Einwirkung eines Oxidationsmittels mit sich selbst oder anderen Oxidationsfarbstoffvorprodukten unter Ausbildung der eigentlichen Farbstoffe reagieren. Dadurch werden hervorragende und haltbare Färbungen erzielt, bei denen auch die Grauabdeckung befriedigend ist. Jedoch kann es aufgrund der verwendeten Oxidationsmittel insbesondere bei häufigerer Anwendung zu einer unerwünschten Beeinträchtigung der Haarstruktur oder sogar zu Schädigungen des Haares kommen.

Aufgabe der Erfindung ist es daher, Haarfärbemittel zur Verfügung zu stellen, mit denen bei der Färbung eine sichere Grauabdeckung erzielt werden kann, ohne das die Haarstruktur in unerwünschter Weise angegriffen wird.

Es wurde nun überraschenderweise gefunden, daß durch Färbemittel auf Basis von Oxidationsfarbstoffvorprodukten die gewünschte Färbung und Grauabdeckung bei gleichzeitiger Schonung der Haarstruktur dann ermöglicht wird, wenn diese Mittel gleichzeitig einen direktziehenden Pflanzenfarbstoff oder das Pflanzenprodukt Henna neutral enthalten.

Gegenstand der Erfindung sind daher feste Haarfärbemittel in Pulver- oder Granulatform, enthaltend mindestens ein Oxidationsfarbstoffvorprodukt, ein Oxidationsmittel sowie übliche kosmetische Zusätze, dadurch gekennzeichnet, daß sie mindestens einen direktziehenden Pflanzenfarbstoff oder Henna neutral enthalten.

Wenngleich die erfindungsgemäßen Produkte insbesondere zum Färben von Haaren geeignet sind, so steht doch ihrem Einsatz auf anderen Färbegebieten, z.B. zur Färbung von Textilfasern oder Wolle, prinzipiell nichts entgegen.

Die erfindungsgemäßen Mittel liegen in Pulver- oder Granulat-Form vor. Sie enthalten als erste zwingende Komponente ein Oxidationsfarbstoffvorprodukt.

Gemäß einer Ausführungsform der Erfindung enthalten die erfindungsgemäßen Mittel nur ein Oxidationsfarbstoffvorprodukt vom Typ der Entwickler.

Beispiele für erfindungsgemäß einzusetzende Entwicklerkomponenten sind primäre aromatische oder heteroaromatische Amine mit einer weiteren, in p-Stellung angeordneten funktionellen Gruppen, wie beispielsweise p-Phenylendiamin, p-Toluylendiamin, p-Aminophenol, 3-Methyl-4-aminophenol, 2-(2-Hydroxyethyl)-1,4-aminobenzol, 2,4,5,6-Tetraminopyrimidin, 2-Hydroxy-4,5,6-triaminopyrimidin, 2,5-Diaminopyridin sowie deren Derivate. Erfindungsgemäß ebenfalls geeignet sind weitere Verbindungen der genannten Art, die zusätzlich eine oder mehrere funktionelle Gruppen wie OH-Gruppen, NH₂-Gruppen, NHR-Gruppen oder NR¹R²-Gruppen aufweisen, in denen R, R¹ und R² unabhängig voneinander für gegebenenfalls substituierte Alkylreste mit 1 bis 4 Kohlenstoffatomen stehen.

Bevorzugt enthalten die erfindungsgemäßen Mittel aber noch mindestens eine Kupplerkomponente. Erfindungsgemäß bevorzugt können auch Mittel sein, die mehr als eine Entwicklerkomponente und mehr als eine Kupplerkomponente enthalten.

Erfindungsgemäß bevorzugte Kupplerkomponenten sind α-Naphthol, Resorcin, 4-Chlorresorcin, 2-Methylresorcin, m-Aminophenol, m-Phenylendiamin, m-Toluylendiamin, 1,5- bzw. 1,7-Dihydroxynaphthalin, 5-Amino-2-methylphenol, 6-Amino-2-methylphenol bzw. Derivate der genannten Verbindungen. Diese können allein oder in Kombination mit weiteren genannten oder anderen, dem Fachmann bekannten, Kupplerkomponenten eingesetzt werden.

Entwickler- und Kupplerkomponenten mit Aminogruppen können auch in Form ihrer Salze, insbesondere der Hydrochloride und der Sulfate, erfindungsgemäß eingesetzt werden.

Eine weitere zwingende Komponente der erfindungsgemäßen Mittel ist das Oxidationsmittel.

Als Oxidationsmittel kommen Additionsverbindungen von Wasserstoffperoxid an Harnstoff, Melamin, Kalium- und Natriumperborat sowie Gemische aus derartigen Wasserstoffperoxid-Anlagerungsverbindungen mit Kalium-, Natrium- oder Ammoniumperoxodisulfat in Betracht.

Kalium- und insbesondere Natriumperborat, allein oder in Kombination mit weiteren Oxidationsmitteln, sind erfindungsgemäß bevorzugt.

Gemäß einer Ausführungsform der Erfindung enthalten die Mittel weiterhin einen direktziehenden Pflanzenfarbstoff. Dieser liegt üblicherweise in pulverförmiger Form vor, die durch Mahlen und nachfolgendes Sieben von verschiedenen Pflanzenteilen wie Stengel, Blüten, Blätter, Wurzeln, Früchte und Samen erhältlich ist. Erfindungsgemäß geeignete Ausgangsstoffe sind beispielsweise Kamille, kleine Kamille-, Hibiskus-, Saflor- und Sonnenblumenblüten, die Blätter des Walnussbaumes, des Hennastrauches, der Indigopflanze, des Sumachs und des Holunders, Rubiaceenwurzeln (Färberröte, Waldmeister, Labkraut), Curcumawurzeln, Lotuswurzeln und Rhabarberwurzeln, rote Sandel-, Campeche-, Brasilien- und Pernambucohölzer, Erlen-, Erdbeerbaum- und Sumarinde, Oleandersamen, Safranblütennarben, Curcuma- und Blutkrautwurzelstöcke, Schlehenfrüchte, Ginsterzweige und ganze Pflanzen von Wau, Flechten und Goldrute.

Besonders bevorzugt als Pflanzenfarbstoffe sind die Henna-Farbstoffe. Rotes Henna besteht aus den getrockneten, gepulverten Blättern der Pflanzen *Lawsonia alba, Lawsonia spinosa* und/oder *Lawsonia inermis.* Sogenanntes Henna schwarz leitet sich von den Blättern der Färberwaid (*Isatis tinctoria*) ab und wird in Mischungen mit *Lawsonia* Henna verwendet, um dunklere Schattierungen im Rotbereich zu erzielen.

Weiterhin bevorzugte Pflanzenfarbstoffe stammen von Walnußschalen, Indigo, Kamille, Blauholz und der Faulbaumrinde.

Alternativ zu den genannten Pflanzenfarbstoffen kann auch Henna neutral eingesetzt werden.

Bei Henna neutral handelt es sich um gepulverte Blätter und/oder gepulverte Rinde von *Cassia auriculata.* Ein entsprechendes Produkt ist aber auch aus den Blättern des Lotosbaumes herstellbar.

Wenngleich in den erfindungsgemäßen Mitteln nur ein Pflanzenfarbstoff oder Henna neutral enthalten sein muß, umfaßt die Erfindung selbstverständlich auch Mittel, die diese beiden Komponenten enthalten.

Insbesondere zur Nuancierung der Färbung können die erfindungsgemäßen Mittel als fakultative Komponente weiterhin synthetische direktziehende Farbstoffe enthalten.

Erfindungsgemäß bevorzugte synthetische direktziehende Farbstoffe sind 4-Hydroxypropylamino-3-nitrophenol, HC Red No. 3, N,N'-Bis-(2-hydroxyethyl)-2-nitro-p-phenylendiamin, HC Blue No. 2, HC Yellow No. 2, HC Yellow No. 12, HC Blue No. 12 und 2-Amino-6-chlor-4-nitrophenol.

Die erfindungsgemäßen Mittel enthalten die Oxidationsfarbstoffvorprodukte bevorzugt in Mengen von 1 - 10 Gew.-%, bezogen auf das gesamte feste Mittel.

Die erfindungsgemäßen Mittel enthalten die Oxidationsmittel bevorzugt in Mengen von 10 - 35 Gew.-%, ebenfalls bezogen auf das gesamte feste Mittel.

Der Gehalt an Pulver von farbstoffhaltigen Pflanzenteilen in den erfindungsgemäßen Haarfärbemitteln kann 3 - 25 Gew.-% betragen. Mengen von 5 - 25 Gew.-%, insbesondere von 5 bis 15 Gew-%, jeweils bezogen auf das gesamte feste Mittel, sind bevorzugt.

Der Gehalt an Henna neutral in den entsprechenden Ausführungsformen der Erfindung beträgt mindestens 25 Gew.-%, bezogen auf das gesamte feste Mittel. Jedoch sollte der Anteil 70 Gew.-% nicht überschreiten. Ein bevorzugter Bereich des Gehaltes an Henna neutral liegt zwischen 40 und 65 Gew.-%, insbesondere zwischen 50 und 60 Gew.-%.

Wenn das erfindungsgemäße Mittel in Pulverform vorliegt, kann je nach Beschaffenheit der einzelnen Komponenten ein unerwünschtes Stauben auftreten. Dies ist nicht nur hinsichtlich der Dosierung problematisch, sondern beispielsweise auch hinsichtlich der Arbeitsbedingungen im Friseurbereich.

Gemäß einer bevorzugten Ausführungsform können daher erfindungsgemäße Mittel einer speziellen Entstaubungsbehandlung unterzogen werden.

Dies geschieht bevorzugt durch Aufsprühen einer inerten organischen Verbindung. Dabei kann es sich sowohl um bei Raumtemperatur flüssige als auch um bei Raumtemperatur feste Verbindungen handeln, vorausgesetzt, letztere lassen sich durch Erwärmen auf mäßig hohe Temperaturen verflüssigen. Üblicherweise wird man lediglich solche feste Verbindungen wählen, deren Schmelzpunkt unterhalb von 50 °C liegt.

Zum Entstauben wird die inerte organische Substanz bevorzugt auf das pulverförmige Haarfärbemittel aufgesprüht.

Als Entstaubungsmittel kommen bevorzugt Paraffine, pflanzliche, tierische und synthetische Öle sowie die niedrig schmelzenden Wachse in Betracht. Weitere geeignete Komponenten sind beispielsweise Triglyceride und Fettalkohole. Besonders bevorzugte Entstaubungsmittel sind Paraffinöle, Paraffine mit Schmelzpunkten im Bereich von 40 °C bis 50 °C, Polysiloxane wie Dimethyl-Polysiloxan, Capryl-Caprinsäure-Triglycerid, Octyldodecanol und Bienenwachs.

Der Anteil der Öle bzw. Wachse in den erfindungsgemäßen Haarfärbemitteln kann zwischen 1- 30 Gew-%, insbesondere zwischen 7 und 15 Gew-%, jeweils berechnet auf die Gesamtzusammensetzung des Haarfärbemittels, betragen.

Die erfindungsgemäßen Haarfärbemittel können als Verdickungsmittel weiterhin Guarmehl, Natriumalginat, Gummi arabicum, Guar- oder Caruba-Gummi, Pektine, Cellulosederivate wie auch Methylcellulose und Hydroxymethylcellulose, Hydroxypropyl-methylcellulose, Carboxymethylcellulose und verschiedene Polymere, insbesondere Acrylsäurederivate, enthalten. Man kann auch anorganische Verdickungsmittel, wie Bentonit und/oder Kieselsäure, verwenden. Diese Verdickungsmittel sind vorzugsweise in Mengen zwischen 0,1 und 5 Gew.-% und insbesondere zwischen 0,5 und 3 Gew.-%, bezogen auf das Gesamtgewicht des Haarfärbemittels, enthalten.

Die Haarfärbemittel können weiterhin kationische und amphotere Polymere, filmbildende anionische und nichtionogene Polymere sowie oberflächenaktive Mittel oder Mischungen davon in Mengen von jeweils 0,1 bis 5 Gew.-% enthalten.

Bevorzugte oberflächenaktive Mittel sind Seifen, Alkylbenzolsulfonate, Alkylnaphthalinsulfonate, Fettalkoholsulfate oder -ethersulfate bzw. -sulfonate, quarternäre Ammoniumverbindungen, Fettsäurediethanolamide, polyethoxylierte oder polyglycerierte Alkylphenole. Die oberflächenaktiven Mittel liegen im erfindungsgemäßen Haarfärbemittel in Anteilen zwischen 0,1 und 25 Gew.-%, vorzugsweise 0,1 und 15 Gew.-%, bezogen auf das Gesamtgewicht des Färbemittels, vor.

Schließlich kann man den erfindungsgemäßen Haarfärbemitteln auch alle anderen herkömmlicherweise in Haarfärbemitteln verwendeten Adjuvantien zusetzen wie insbesondere Penetrationsmittel, Sesquestriermittel, Antioxidantien, Puffer und Parfums.

Gegenstand der Erfindung ist auch ein Verfahren zur Herstellung eines Haarfärbemittels in Pulver- oder Granulatform, bei dem das Oxidationsfarbstoffvorprodukt, das Oxidationsmittel, der Pflanzenfarbstoff und/oder das Henna neutral gegebenenfalls unter Zugabe von in Haarfärbemitteln üblichen Zusätzen gut durchmischt werden und die erhaltene Pulvermischung unter ständiger Bewegung mit einer inerten organischen Verbindung, die gegebenenfalls durch Erhitzen verflüssigt wurde, besprüht wird und das erhaltene Pulvergemisch gegebenenfalls in die Granulatform überführt wird.

Aus den erfindungsgemäßen Mitteln wird unmittelbar vor der Anwendung durch Zugabe entsprechender Mengen von Wasser ein Färbebrei hergestellt, der im weiteren als Anwendungsmischung bezeichnet wird.

Bevorzugte Anwendungsmischungen bestehen aus 15 - 25 Gew.-% eines erfindungsgemäßen Färbemittels und 75 - 85 Gew.-% Wasser. Sie sind durch entsprechende Zugabe von Säuren oder Basen, die in einer bevorzugten Ausführungsform bereits im Färbemittel enthalten sind, üblicherweise auf einen pH-Wert von 5,0 bis 12,0 eingestellt. Dazu können organische oder anorganische Säuren, Hydroxide, Alkali- oder Erdalkalicarbonaten oder Alkalisilikaten verwendet werden.

Die Anwendungsmischung kann sowohl auf feuchtes als auch auf trockenes Haar aufgebracht werden. Das Aufbringen auf trockenes Haar kann erfindungsgemäß bevorzugt sein.

### Beispiele

### 1. Anwendung erfindungsgemäßer Mittel

Es wurde eine Anwendungsmischung aus 80 g Haarfärbemittel der Erfindung und 320 ml heißem Wasser in Form eines streichfähigen Breies bereitgestellt. Dieser Haarfärbemittelbrei wurde sofort mit einem Pinsel auf das trockene, vorher nicht gewaschene, schulterlange Haar zügig und schnell vom Haaransatz bis zur Spitze satt aufgelegt. Das feine Schläfenhaar wurde zuletzt nur dünn versorgt. Es war darauf zu achten, daß das gesamte Haar gut mit dem Haarfärbemittelbrei umhüllt war. Danach wurde das gesamte Haar mit einer Haube so bedeckt, daß diese an den Konturen eng anlag und so gebunden war, daß sie nicht verrutschen konnte. Der aufbetragene Brei wurde auf dem so abgedeckten Haar 30 Minuten unter Wärmezufuhr (ca. 40°C) belassen. Nach dieser Einwirkzeit wurde die Haube abgenommem und der Haarfärbemittelbrei mit Leitungswasser (30°C) gründlich ausgeschwemmt. Danach wurde das Haar shampooniert und gespült, bis Haar und Haut völlig sauber vorlagen.

### 2. Formulierungsbeispiele

### 2.1. Haarfärbemittel in Pulverform

0,10 kg Resorcin
0,10 kg 1-Hydroxy-3-aminobenzol
0,15 kg 2-Amino-3-hydroxypyridin
0,10 kg 4-Hydroxypropylamino-3-nitrophenol
0,15 kg HC Yellow No.12
0,35 kg 2-Methylresorcin
1,20 kg 2,5-Diaminotoluolsulfat
1,00 kg Indigo
2,00 kg Walnußschalen
4,00 kg Henna rot
54,85 kg Henna neutral
23,00 kg Natriumperborat
5,00kg Carboxymethylcellulose

92,00 kg der vorstehenden Mischung wurden in einem Mischer (zum Beispiel einem Lödige-Mischer) zusammengerührt und anschließend mit 8,00 kg Paraffinöl (35 mPa*s, gemessen bei 20 °C) besprüht.

40 g des Haarfärbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf hellbraunes Haar mit einem Grauanteil von 50% einwirken. Danach wurde der Brei ausgespült und das Haar shampooniert und getrocknet. Das hellbraune Ausgangshaar mit einem Grauanteil von 50% zeigte einen einheitlichen hellen Mokkabraunton.

### 2.2. Haarfärbemittel in Pulverform

0,15 kg Resorcin
0,10 kg 1-Hydroxy-3-aminobenzol
0,15 kg 2-Amino-3-hydroxypyridin
0,15 kg 4-Hydroxypropylamino-3-nitrophenol
0,10 kg HC Yellow No.12
0,30 kg 2-Methylresorcin
1,25 kg 2,5-Diaminotoluolsulfat
1,00 kg Kamille
1,50 kg Henna schwarz
2,50 kg Henna rot
60,80 kg Henna neutral
20,00 kg Natriumperborat
5,00kg Carboxymethylcellulose

93,00 kg der vorstehenden Mischung wurden in einem Mischer (zum Beispiel einem Lödige-Mischer) zusammengerührt und anschließend mit 7,00 kg Paraffinöl (15 mPa*s, gemessen bei 20 °C) besprüht.

40 g des Haarfärbemittels wurden mit 160 g warmen Wassers (40°-50°C) gut vermischt, um eine gut streichfähige Anwendungsmischung zu erhalten. Dieses Gemisch, mit einem Pinsel aufgetragen, ließ man 30 Minuten bei 40°C auf dunkelblondes Haar mit einem Grauanteil von 50% einwirken. Danach wurde die Anwendungsmischung ausgespült, und das Haar shampooniert und getrocknet. Das dunkelblonde Ausgangshaar mit einem Grauanteil von 50% zeigte nach der Behandlung einen einheitlichen hellen Kastanienton.

### 2.3. Haarfärbemittel in Pulverform

0,30 kg Resorcin
0,10 kg 1-Hydroxy-3-aminobenzol
0,15 kg 2-Amino-3-hydroxypyridin
0,15 kg HC Yellow No. 12
0,45 kg 2-Methylresorcin
2,00 kg 2,5-Diaminotoluolsulfat
1,50 kg Kamille
2,00 kg Faulbaumrinde
3,50 kg Henna rot
52,85 kg Henna neutral
22,00 kg Natriumperborat
5,00kg Carboxymethylcellulose

90,00 kg der vorstehenden Mischung wurden in einem Mischer (zum Beispiel einem Lödige-Mischer) zusammengerührt und anschließend mit 10,00 kg Capryl-Caprinsäure-Triglycerid besprüht.

40 g des Haarfärbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf mittelbraunes Haar mit einem Grauanteil von 50% einwirken. Danach wurde der Brei ausgespült und das Haar shampooniert und getrocknet. Das mittelbraune Ausgangshaar mit einem Grauanteil von 50% wies nach der Behandlung einen einheitlichen dunklen Mokkabraunton auf.

### 3.4. Haarfärbemittel in Pulverform

0,35 kg Resorcin
0,10 kg 1-Hydroxy-3-aminobenzol
0,25 kg 2-Amino-3-hydroxypyridin
0,30 kg 4-Hydroxypropylamino-3-nitrophenol
0,10 kg HC Yellow No.12
0,45 kg 2-Methylresorcin
2,25 kg 2,5-Diaminotoluolsulfat
1,00 kg Blauholz
1,00 kg Indigo
1,00 kg Walnußschalen
57,20 kg Henna neutral
22,00 kg Natriumperborat
5,00 kg Carboxymethylcellulose

91,00 kg der vorstehenden Mischung wurden in einem Mischer (zum Beispiel einem Lödige-Mischer) zusammengerührt und anschließend mit 9,00 kg Octyldodecanol besprüht.

40 g des Haarfarbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf hellbraunes Haar mit einem Grauanteil von 50% einwirken. Danach spülte man den Brei aus und shampoonierte und trocknete das Haar. Das hellbraune Ausgangshaar mit einem Grauanteil von 50% wies nun einen einheitlichen dunklen Kastanienton auf.

### 3.5. Haarfärbemittel in Pulverform

0,20 kg Resorcin
0,15 kg 1-Hydroxy-3-aminobenzol
0,01 kg 4-Hydroxypropylamino-3-nitrophenol
0,05 kg 2-Amino-6-chlor-4-nitrophenol
0,40 kg 2-Methylresorcin
0,90 kg 2,5-Diaminotoluol Sulfat
1,50 kg Blauholz
1,00 kg Kamille
6,00 kg Henna rot
56,29 kg Henna neutral
21,00 kg Natriumperborat
5,00kg Carboxymethylcellulose

92,50 kg der vorstehenden Mischung wurden in einem Mischer (zum Beispiel einem Lödige-Mischer) zusammengerührt und anschließend mit 7,50 kg Dimethyl-Polysiloxan besprüht.

40 g des Haarfärbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf mittelblondes Haar mit einem Grauanteil von 80% einwirken. Danach spülte man den Haarfärbemittelbrei aus, shampoonierte und trocknete das Haar. Das mittelblonde Ausgangshaar mit einem Grauanteil von 80% lag nach der Behandlung in einem einheitlichen Mittelblondton vor.

### 3.6. Haarfärbemittel in Pulverform

0,10 kg Resorcin
0,10 kg 1-Hydroxy-3-aminobenzol
0,15 kg 2-Amino-3-hydroxypyridin
0,10 kg 4-Hydroxypropylamino-3-nitrophenol
0,15 kg HC Yellow No.12
0,35 kg 2-Methylresorcin
1,20 kg 2,5-Diaminotoluolsulfat
61,85 kg Henna neutral
23,00 kg Natriumperborat
5,00kg Carboxymethylcellulose

92,00 kg der vorstehenden Mischung wurden in einem Mischer (zum Beispiel einem Lödige-Mischer) zusammengerührt und anschließend mit 8,00 kg Paraffinöl (35 mPa*s, gemessen bei 20 °C) besprüht.

40 g des Haarfärbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf hellbraunes Haar mit einem Grauanteil von 50% einwirken. Danach spülte man den Brei aus, shampoonierte und trocknete das Haar. Das hellbraune Ausgangshaar mit einem Grauanteil von 50% zeigte einen einheitlichen hellen Tabakbraunton.

### 3.7. Haarfärbemittel in Pulverform

0,15 kg Resorcin
0,10 kg 1-Hydroxy-3-aminobenzol
0,15 kg 2-Amino-3-hydroxypyridin
0,15 kg 4-Hydroxypropylamino-3-nitrophenol
0,10 kg HC Yellow No.12
0,30 kg 2-Methylresorcin
1,25 kg 2,5-Diaminotoluolsulfat
65,80 kg Henna neutral
20,00 kg Natriumperborat
5,00kg Carboxymethylcellulose

93,00 kg der vorstehenden Mischung wurden in einem Mischer (zum Beispiel einem Lödige-Mischer) zusammengerührt und anschließend mit 7,00 kg Paraffinöl (15 mPa*s, gemessen bei 20 °C) besprüht.

40 g des Haarfärbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf mittelblondes Haar mit einem Grauanteil von 50% einwirken. Danach spülte man den Brei aus, shampoonierte und trocknete das Haar. Das mittelblonde Ausgangshaar mit einem Grauanteil von 50% zeigte nach der Behandlung einen einheitlichen Kastanienton.

### 3.8. Haarfärbemittel in Pulverform

0,30 kg Resorcin
0,10 kg 1-Hydroxy-3-aminobenzol
0,15 kg 2-Amino-3-hydroxypyridin
0,15 kg HC Yellow No. 12
0,45 kg 2-Methylresorcin
2,00 kg 2,5-Diaminotoluolsulfat
59,85 kg Henna neutral
22,00 kg Natriumperborat
5,00kg Carboxymethylcellulose

90,00 kg der vorstehenden Mischung wurden in einem Mischer (zum Beispiel einem Lödige-Mischer) zusammengerührt und anschließend mit 10,00 kg Capryl-Caprinsäure-Triglycerid besprüht.

40 g des Haarfärbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf mittelbraunes Haar mit einem Grauanteil von 50% einwirken. Danach spülte man den Brei aus, shampoonierte und trocknete das Haar. Das mittelbraune Ausgangshaar mit einem Grauanteil von 50% weist nach der Behandlung einen einheitlichen dunklen Kakaoton auf.

### 3.9. Haarfärbemittel in Pulverform

0,50 kg Resorcin
0,20 kg 1-Hydroxy-3-aminobenzol
0,10 kg 2-Amino-3-hydroxypyridin
0,45 kg 2-Methylresorcin
2,25 kg 2,5-Diaminotoluol Sulfat
60,50 kg Henna neutral
22,00 kg Natriumperborat
5,00kg Carboxymethylcellulose

91,00 kg der vorstehenden Mischung wurden in einem Mischer (zum Beispiel einem Lödige-Mischer) zusammengerührt und anschließend mit 9,00 kg Octyldodecanol besprüht.

40 g des Haarfärbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf hellbraunes Haar mit einem Grauanteil von 50% einwirken. Danach spülte man den Brei aus, shampoonierte und trocknete das Haar. Das hellbraune Ausgangshaar mit einem Grauanteil von 50% wies einen einheitlichen Mittelbraunton auf.

### 3.10. Haarfärbemittel in Pulverform

0,20 kg Resorcin
0,15 kg 1-Hydroxy-3-aminobenzol
0,01 kg 4-Hydroxypropylamino-3-nitrophenol
0,05 kg 2-Amino-6-chlor-4-nitrophenol
0,40 kg 2-Methylresorcin
0,90 kg 2,5-Diaminotoluolsulfat
64,79 kg Henna neutral
21,00 kg Natriumperborat
5,00kg Carboxymethylcellulose

92,50 kg der vorstehenden Mischung wurden in einem Mischer (zum Beispiel einem Lödige-Mischer) zusammengerührt und anschließend mit 7,50 kg Dimethyl-Polysiloxan besprüht.

40 g des Haarfärbemittels wurden mit 160 g warmen Wassers (40°-50°C) gut vermischt, um einen gut streichfähigen Brei zu erhalten. Dieses Gemisch, mit einem Pinsel aufgetragen, ließ man 30 Minuten bei 40°C auf hellblondes Haar mit einem Grauanteil von 80% einwirken. Danach spülte man den Brei aus, shampoonierte und trocknete das Haar. Das hellblonde Ausgangshaar mit einem Grauanteil von 80% lag nach der Behandlung in einem einheitlichen Mittelblondton vor.

Feste Haarfärbemittel in Pulver- oder Granulatform, enthaltend mindestens ein Oxidationsfarbstoffvorprodukt, ein Oxidationsmittel sowie übliche kosmetische Zusätze, die zusätzlich mindestens einen direktziehenden Pflanzenfarbstoff oder Henna neutral enthalten, zeichnen sich durch exzellente Färbeergebnisse mit vollständiger Grauabdeckung unter hoher Schonung des Haares aus. Die Mittel in Form von Anwendungsmischungen auf das Haar aufgebracht, die durch Vermischen der Mittel mit Wasser bereitet werden.

## Patentansprüche

1. Festes Haarfärbemittel in Pulver- oder Granulatform, enthaltend mindestens ein Oxidationsfarbstoffvorprodukt, ein Oxidationsmittel sowie übliche kosmetische Zusätze, **dadurch gekennzeichnet, daß** es mindestens einen direktziehenden Pflanzenfarbstoff oder Henna neutral enthält.

2. Mittel nach Anspruch 1, **dadurch gekennzeichnet, daß** es als Oxidationsfarbstoffvorprodukte mindestens eine Entwicklerkomponente und mindestens eine Kupplerkomponente enthält.

3. Mittel nach Anspruch 1 oder 2, **dadurch gekennzeichnet, daß** die Entwicklerkomponente ausgewählt ist aus p-Phenylendiamin, p-Toluylendiamin, 2-(2-Hydroxyethyl)-1,4-diaminobenzol, p-Aminophenol, 3-Methyl-4-aminophenol, 2,4,5,6-Tetraaminopyrimidin und 2-Hydroxy-4,5,6-Triaminopyrimidin.

4. Mittel nach Anspruch 2 oder 3, **dadurch gekennzeichnet, daß** es als Kupplersubstanz α-Naphthol, Resorcin, 4-Chlorresorcin, 2-Methylresorcin, m-Aminophenol, m-Phenylendiamin, m-Toluylendiamin, 1,5- bzw. 1,7-Dihydroxynaphthalin, 5-Amino-2-methylphenol oder 6-Amino-2-methylphenol enthält.

5. Mittel nach einem der Ansprüche 1 bis 4, **dadurch gekennzeichnet, daß** das Oxidationsmittel Natriumperborat ist.

6. Mittel nach einem der Ansprüche 1 bis 5, **dadurch gekennzeichnet, daß** der Pflanzenfarbstoff ausgewählt ist aus der Gruppe, die Henna rot, Henna schwarz, Walnußschalen, Indigo, Kamille, Blauholz und Faulbaumrinde umfaßt.

7. Mittel nach einem der Ansprüche 1 bis 6, **dadurch gekennzeichnet, daß** es weiterhin einen synthetischen direktziehenden Farbstoff enthält.

8. Mittel nach Anspruch 7, **dadurch gekennzeichnet, daß** der direktziehende synthetische Farbstoff ausgewählt ist aus 4-Hydroxypropylamino-3-nitrophenol, HC Red No. 3, N,N'-Bis-(2-hydroxyethyl)-2-nitro-p-phenylendiamin, HC Blue No. 2, HC Yellow No. 2, HC Yellow No. 12, HC Blue No. 12 und 2-Amino-6-chlor-4-nitrophenol.

9. Mittel nach einem der Ansprüche 1 bis 8, **dadurch gekennzeichnet, daß** der Gehalt an Oxidationsfarbstoffvorprodukten 1 - 10 Gew.-%, bezogen auf das feste Mittel, beträgt.

10. Mittel nach einem der Ansprüche 1 bis 9, **dadurch gekennzeichnet, daß** der Gehalt an Oxidationsmittel 10 - 35 Gew.-%, bezogen auf das feste Mittel, beträgt.

11. Mittel nach einem der Ansprüche 1 bis 10, **dadurch gekennzeichnet, daß** der Gehalt an Pflanzenfarbstoffen 5 - 20 Gew.-%, bezogen auf das feste Mittel, beträgt.

12. Mittel nach einem der Ansprüche 1 bis 11, **dadurch gekennzeichnet, daß** der Gehalt an Henna neutral 40 - 65 Gew.-%, insbesondere 50 - 60 Gew.-%, beträgt.

13. Mittel nach einem der Ansprüche 1 bis 12, **dadurch gekennzeichnet, daß** es 1 - 30 Gew.-%, bezogen auf das feste Mittel, einer inerten organischen Verbindung mit einem Schmelzpunkt unterhalb von 50 °C enthält.

14. Mittel nach Anspruch 13, **dadurch gekennzeichnet, daß** die inerte organische Verbindungen ausgewählt ist aus Paraffinöl, Triglyceriden, Fettalkoholen und Polysiloxanen.

15. Anwendungsmischung zum Färben von Haaren, **dadurch gekennzeichnet, daß** sie aus 15 - 25 Gew.-% eines Haarfärbemittels gemäß einem der Ansprüche 1 bis 14 und 85 - 75 Gew.-% Wasser enthält.

16. Anwendungsmischung nach Anspruch 15, **dadurch gekennzeichnet, daß** sie einen pH-Wert von 5,0 bis 12,0 aufweist.

17. Verfahren zur Herstellung eines Haarfärbemittels in Pulver- oder Granulatform nach einem der Ansprüche 1 bis 14, bei dem das Oxidationsfarbstoffvorprodukt, das Oxidationsmittel, der Pflanzenfarbstoff und/oder das Henna neutral gegebenenfalls unter Zugabe von in Haarfärbemitteln üblichen Zusätzen gut durchmischt werden und die erhaltene Pulvermischung unter ständiger Bewegung mit einer inerten organischen Verbindung, die gegebenenfalls durch Erhitzen verflüssigt wurde, besprüht wird und das erhaltene Pulvergemisch gegebenenfalls in die Granulatform überführt wird.

## Claims

1. A solid hair colorant in powder or granular form containing at least one oxidation dye precursor, an oxidizing agent and typical cosmetic additives, **characterized in that** it contains at least one substantive plant dye or henna neutral.

2. A hair colorant as claimed in claim 1, **characterized in that** it contains at least one primary intermediate and at least one secondary intermediate as oxidation dye precursors.

3. A hair colorant as claimed in claim 1 or 2, **characterized in that** the primary intermediate is selected from p-phenylenediamine, p-toluylene-diamine, 2-(2-hydroxyethyl)-1,4-diaminobenzene, p-aminophenol, 3-methyl-4-aminophenol, 2,4,5,6-tetraaminopyrimidine and 2-hydroxy-4,5,6-triaminopyrimidine.

4. A hair colorant as claimed in claim 2 or 3, **characterized in that** it contains α-naphthol, resorcinol, 4-chlororesorcinol, 2-methyl resorcinol, m-aminophenol, m-phenylenediamine, m-toluylenediamine, 1,5- or 1,7-dihydroxynaphthalene, 5-amino-2-methylphenol or 6-amino-2-methylphenol as secondary intermediate.

5. A hair colorant as claimed in any of claims 1 to 4, **characterized in that** the oxidizing agent is sodium perborate.

6. A hair colorant as claimed in any of claims 1 to 5, **characterized in that** the plant dye is selected from the group consisting of henna red, henna black, walnut shells, indigo, camomile, logwood and black alder.

7. A hair colorant as claimed in any of claims 1 to 6, **characterized in that** it additionally contains a synthetic substantive dye.

8. A hair colorant as claimed in claim 7, **characterized in that** the substantive synthetic dye is selected from 4-hydroxypropylamino-3-nitrophenol, HC Red No. 3, N,N'-bis-(2-hydroxyethyl)-2-nitro-p-phenylenediamine, HC Blue No. 2, HC Yellow No. 2, HC Yellow No. 12, HC Blue No. 12 and 2-amino-6-chloro-4-nitrophenol.

9. A hair colorant as claimed in any of claims 1 to 8, **characterized in that** it contains from 1 to 10% by weight of oxidation dye precursors, based on the solid hair colorant.

10. A hair colorant as claimed in any of claims 1 to 9, **characterized in that** it contains 10 to 35% by weight of oxidizing agent, based on the solid hair colorant.

11. A hair colorant as claimed in any of claims 1 to 10, **characterized in that** it contains 5 to 20% by weight of plant dyes, based on the solid hair colorant.

12. A hair colorant as claimed in any of claims 1 to 11, **characterized in that** it contains 40 to 65% by weight and more particularly 50 to 60% by weight of henna neutral.

13. A hair colorant as claimed in any of claims 1 to 12, **characterized in that** it contains 1 to 30% by weight, based on the solid hair colorant, of an inert organic compound with a melting point below 50°C.

14. A hair colorant as claimed in claim 13, **characterized in that** the inert organic compound is selected from paraffin oil, triglycerides, fatty alcohols and polysiloxanes.

15. An application mixture for coloring hair, **characterized in that** it contains 15 to 25% by weight of the hair colorant claimed in any of claims 1 to 14 and 85 to 75% by weight of water.

16. An application mixture as claimed in claim 15, **characterized in that** a pH of 5.0 to 12.0.

17. A process for the production of the powder-form or granular hair colorant claimed in any of claims 1 to 14, **characterized in that** the oxidation dye precursor, the oxidizing agent, the plant dye and/or the henna neutral are thoroughly mixed, optionally with addition of additives typically used in hair colorants, an inert organic compound optionally liquefied by heating is sprayed onto the powder mixture obtained with continuous agitation and the powder mixture is optionally converted into granular form.

## Revendications

1. Colorant solide pour cheveux sous forme de poudre ou de granulé contenant au moins un produit précurseur de colorant par oxydation, un agent oxydant ainsi que des additifs usuels cosmétiques,
**caractérisé en ce qu'**
il contient au moins un colorant végétal ou du henné neutre.

2. Colorant selon la revendication 1,
**caractérisé en ce qu'**
il contient comme produits précurseurs de colorants par oxydation un composant de développeur et au moins un composant de coupleur.

3. Colorant selon l'une quelconque des revendications 1 ou 2,
**caractérisé en ce que**
le composant de développeur est choisi parmi la p-phénylènediamine. la p-tolulènediamine, le 2-(2-hydroxyéthyl)-1,4-diaminobenzène, le p-aminophénol, le 3-méthyl-4-aminophénol, la 2,4,5,6-tétraaminopyrimidine et la 2-hydroxy-4,5,6-triaminopyrimidine.

4. Colorant selon l'une quelconque des revendications 2 ou 3,
**caractérisé en ce qu'**
il contient comme substance de coupleur de l'α-naphtol, le resorcinol, le 4-chlororésorcinol, le 2-méthylrésorcinol, le m-aminophénol, la m-phenylènediamine, la m-toluylènediamine, le 1,5- ou le 1,7-dihydroxynaphtalène, le 5-amino-2-méthylphénol, le 6-amino-2-méthylphénol.

5. Colorant selon l'une quelconque des revendications 1 à 4,
**caractérisé en ce que**
l'agent oxydant est le perborate de sodium.

6. Colorant selon l'une quelconque des revendications 1 à 5,
**caractérisé en ce que**
le colorant végétal est choisi dans le groupe qui comprend le henné rouge, le henné noir, les coques de noix, l'indigo, la camomille, le bois de campêche et les écorces de bourdaine.

7. Colorant selon l'une quelconque des revendications 1 à 6,
**caractérisé en ce qu'**
il contient en outre un colorant synthétique à action directe.

8. Colorant selon la revendication 7,
**caractérisé en ce que**
le colorant synthétique à action directe est choisi parmi le 4-Hydroxypropylamino-3-nitrophénol, HC Red No. 3, la N,N'-bis(2-hydroxyéthyl)-2-nitro-p-phénylènedlamine, HC Blue No. 2, HC Yellow No. 2, HC Yellow No. 12, HC Blue No. 12 et le 2-amino-6-chloro-4-nitrophénol.

9. Colorant selon l'une quelconque des revendications 1 à 8
**caractérisé en ce que**
la teneur en produits précurseurs de colorant par oxydation s'élève de 1 à 10 % en poids rapporté au produit solide.

10. Colorant selon l'une quelconque des revendications 1 à 9
**caractérisé en ce que**
la teneur en agent oxydant s'élève de 10 à 35 % en poids rapporté au produit solide.

11. Colorant selon l'une quelconque des revendications 1 à 10
**caractérisé en ce que**
la teneur en colorants végétaux de 5à 20 en poids rapporté au produit solide.

12. Colorant selon l'une quelconque des revendications 1 à 11
**caractérisé en ce que**
la teneur en henné neutre s'élève de 40 à 65 % en poids en particulier de 50 à 60 % en poids.

13. Colorant selon l'une quelconque des revendications 1 à 12
**caractérisé en ce qu'**
il contient de 1 à 30 % en poids rapporté au produit solide un composé organique inerte ayant un point de fusion en dessous de 50°C.

14. Colorant selon la revendication 13,
**caractérisé en ce que**
les composée organiques inertes sont choisis parmi l'huile de paraffine, les triglycérides, les alcools gras et les polysiloxanes.

15. Mélange d'application pour la teinture des cheveux,
**caractérisé en ce qu'**
il contient de 15 à 25 % en poids d'un colorant pour les cheveux conformément à l'une des revendications 1 à 14 et de 85 à 75 % en poids d'eau.

16. Mélange d'application selon la revendication 15,
**caractérisé en ce qu'**
il possède une valeur de pH de 5,0 à 12,0.

17. Procédé de préparation d'un colorant pour cheveux sous forme de poudre ou de granulé, selon l'une quelconque des revendications 1 à 14, dans lequel le produit précurseur de colorant par oxydation, l'agent oxydant, le colorant végétal et/ou le henné neutre, est bien mélangé le cas échéant en ajoutant des additifs usuels dans les colorants pour cheveux, et le mélange de poudres obtenu est aspergé avec mouvement permanent avec un composé organique inerte qui le cas échéant a été rendu liquide par chauffage, et le mélange de poudres obtenu est converti le cas échéant sous la forme de granulé.
